# EUROPEAN PATENT APPLICATION

(11) **EP 4 737 440 A1**
(43) Date of publication of application: **06.05.2026**
(21) Application number: 25208948.7
(22) Date of filing: 15.10.2025
(51) Int. Cl.: C07D 209/30

(54) **METHOD FOR PREPARING FMOC-TRP(BOC)-OH**

(30) Priority: 29.10.2024 CN 202411524702
(71) Applicant: Sichuan Shifang Sangao Biochemical Industrial Co., Ltd., Chengdu, Sichuan 618415 (CN)
(72) Inventor: FANG, Xiaolong, Chengdu, 618415 (CN); DENG, Jianlong, Chengdu, 618415 (CN); LIANG, Hongguo, Chengdu, 618415 (CN); LIU, Runyu, Chengdu, 618415 (CN); LIN, Shiyu, Chengdu, 618415 (CN); FENG, Xubin, Chengdu, 618415 (CN); LI, Jiaxun, Chengdu, 618415 (CN)
(74) Representative: V.O.

(57) **Abstract**

The present application provides a method for preparing Fmoc-Trp(Boc)-OH, comprising steps of: reacting tryptophan with N-(9-fluorenylmethoxycarbonyloxy)succinimide in the presence of a base to obtain an intermediate compound as represented by formula (3), reacting the intermediate compound as represented by formula (3) with benzyl halide in the presence of a base to obtain an intermediate compound as represented by formula (4); reacting the intermediate compound as represented by formula (4) with di-tert-butyl dicarbonate in the presence of a catalyst to obtain an intermediate compound as represented by formula (6), deprotecting the intermediate compound as represented by formula (6) to obtain Fmoc-Trp(Boc)-OH. The method provided in the present application uses a four-step reaction to synthesize Fmoc-Trp(Boc)-OH, avoiding a step of introducing Fmoc on H-Trp(Boc)-OH. The method simplifies the process, features simple operations and mild reaction conditions, and reduces the difficulty of industrial production. Furthermore, the method does not involve expensive raw materials, which is beneficial for controlling production costs, and the product can be obtained with a high purity and a high yield.

## Description

### FIELD

The present disclosure relates to the field of pharmaceutical synthesis technology, and more specifically relates to a method for preparing Fmoc-Trp(Boc)-OH.

### BACKGROUND

Fmoc-Trp(Boc)-OH is a common intermediate in the synthesis of peptides such as semaglutide, tirzepatide, and the like. The full chemical name of Fmoc-Trp(Boc)-OH is N-alpha-(9-fluorenylmethoxycarbonyl)-N-in-tert-butoxycarbonyl-L-tryptophan, with a molecular formula of C₃₁H₃₀N₂O₆, a CAS number of 143824-78-6, and a structural formula of:

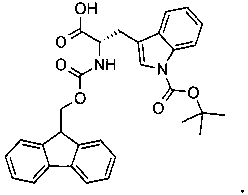

In the synthetic routes of Fmoc-Trp(Boc)-OH disclosed in the prior art, an intermediate H-Trp(Boc)-OH is always obtained first, then Fmoc is introduced to obtain Fmoc-Trp(Boc)-OH. However, H-Trp(Boc)-OH is unstable during the step of introducing Fmoc, resulting in production of Fmoc-Trp-OH as an impurity, which is almost impossible to be removed from Fmoc-Trp(Boc)-OH, thereby influencing the yield and purity of Fmoc-Trp(Boc)-OH.

For example, CN202110672717 has disclosed two routes for synthesizing Fmoc-Trp(Boc)-OH.

The first route is:

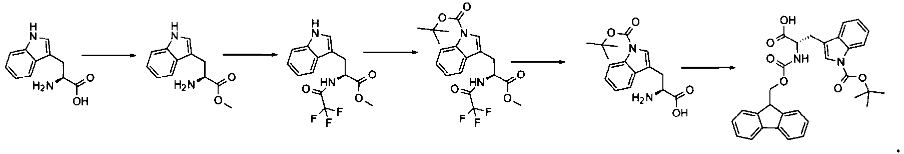

The second route is:

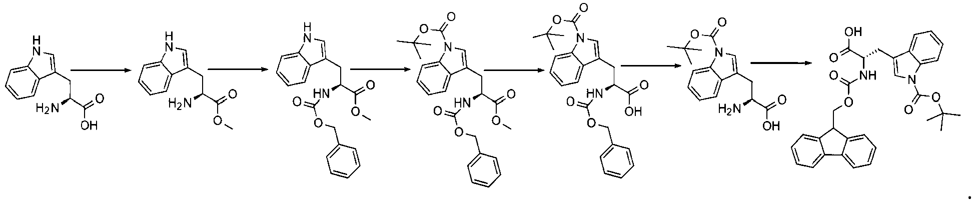

CN202211006819 has also disclosed a synthesis route as follows:

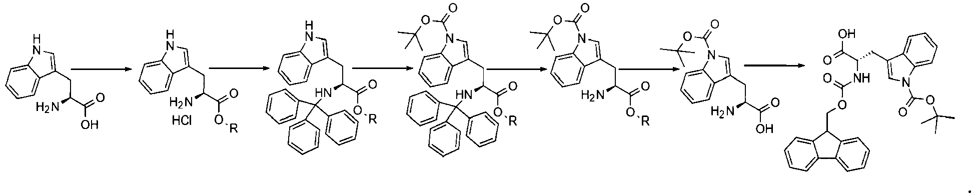

The aforementioned synthesis routes have a relatively low yield and purity, as well as a complex process.

### SUMMARY

In view of the foregoing, the present disclosure provides a method for preparing Fmoc-Trp(Boc)-OH. The method provided in the present disclosure features a simple process, a mild reaction condition, and Fmoc-Trp(Boc)-OH can be obtained with a high purity and a high yield.

The present disclosure provides a method for preparing Fmoc-Trp(Boc)-OH, comprising steps of:
a) reacting tryptophan with N-(9-fluorenylmethoxycarbonyloxy)succinimide in the presence of a base to obtain an intermediate compound as represented by formula (3); 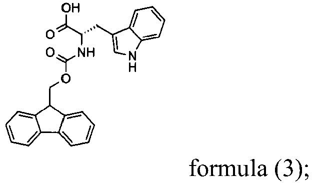
b) reacting the intermediate compound as represented by formula (3) with benzyl halide in the presence of a base to obtain an intermediate compound as represented by formula (4); 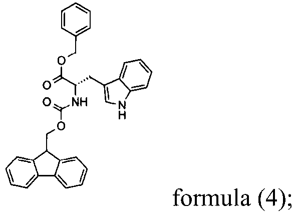
c) reacting the intermediate compound as represented by formula (4) with di-tert-butyl dicarbonate in the presence of a catalyst to obtain an intermediate compound as represented by formula (6); and 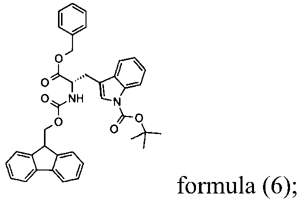
d) deprotecting the intermediate compound as represented by formula (6) to obtain Fmoc-Trp(Boc)-OH.

In some specific embodiments, in step a), the base is an inorganic base; and
tryptophan, N-(9-fluorenylmethoxycarbonyloxy)succinimide, and the base have a mole ratio of 1:(0.8 to 1.3):(1 to 2.5).

In some specific embodiments, in step a), the reaction is performed at 25°C to 35°C for 2 h to 6 h; and
the reaction is performed in a medium, and the medium is water and an organic solvent, wherein the organic solvent is selected from the group consisting of ethyl acetate, acetone, and tetrahydrofuran.

In some specific embodiments, in step b), the base is an inorganic base; and
the intermediate compound as represented by formula (3), benzyl halide, and the base have a mole ratio of 1:(1 to 1.5):(1.1 to 2.0).

In some specific embodiments, in step b), the base is selected from the group consisting of sodium carbonate and potassium carbonate.

In some specific embodiments, in step b), the reaction is performed at 60°C to 80°C for 2 h to 4 h; and
the reaction is performed in a medium, and the medium is an organic solvent, wherein the organic solvent is selected from the group consisting of acetonitrile and ethyl acetate.

In some specific embodiments, in step c), the catalyst is 4-dimethylaminopyridine; and
the intermediate compound as represented by formula (4), the catalyst, and di-tert-butyl dicarbonate have a mole ratio of 1:(0.040 to 0.045):(1 to 2).

In some specific embodiments, in step c), the reaction is performed at 10°C to 35°C for 0.5 h to 2 h; and
the reaction is performed in a medium, and the medium is an organic solvent, wherein the organic solvent is selected from the group consisting of dichloromethane, acetonitrile, ethyl acetate, and tetrahydrofuran.

In some specific embodiments, step d) specifically comprises:
reacting the intermediate compound as represented by formula (6) with hydrogen in the presence of a catalyst to obtain Fmoc-Trp(Boc)-OH.

In some specific embodiments, in step d), the catalyst is selected from the group consisting of Pd/C, raney nickel, and Pd/BaSO4;
the intermediate compound as represented by formula (6) and the catalyst have a mass ratio of 1:(0.01 to 0.1); and
the reaction is performed at 20°C to 30°C for 16 h to 20 h.

The present disclosure provides a method for preparing Fmoc-Trp(Boc)-OH, comprising steps of: a) reacting tryptophan with N-(9-fluorenylmethoxycarbonyloxy)succinimide in the presence of a base to obtain an intermediate compound as represented by formula (3); b) reacting the intermediate compound as represented by formula (3) with benzyl halide in the presence of a base to obtain an intermediate compound as represented by formula (4); c) reacting the intermediate compound as represented by formula (4) with di-tert-butyl dicarbonate in the presence of a catalyst to obtain an intermediate compound as represented by formula (6); d) and deprotecting the intermediate compound as represented by formula (6) to obtain Fmoc-Trp(Boc)-OH. The method provided in the present disclosure uses tryptophan (H-Trp-OH) as a starting material to synthesize Fmoc-Trp(Boc)-OH via a four-step reaction comprising a Fmoc introduction reaction, an esterification reaction with benzyl halide, a Boc introduction reaction, and a hydrogenation reaction, avoiding a step of introducing Fmoc on H-Trp(Boc)-OH. The method simplifies the process, features simple operations and mild reaction conditions, and reduces the difficulty of industrial production. Furthermore, the method does not involve expensive raw materials, which is beneficial for controlling production costs, and Fmoc-Trp(Boc)-OH can be obtained with a high purity and a high yield. Experimental results show that Fmoc-Trp(Boc)-OH obtained by the method provided in the present disclosure has a purity over 98.0% and a yield over 75%.

### BRIEF DESCRIPTION OF DRAWINGS

FIG. 1 is an HPLC chromatogram of intermediate Fmoc-Trp-OH prepared in Example 1;
FIG. 2 is an HPLC chromatogram of intermediate Fmoc-Trp-OBZl prepared in Example 1;
FIG. 3 is an HPLC chromatogram of intermediate Fmoc-Trp(Boc)-OBZl prepared in Example 1;
FIG. 4 is an HPLC chromatogram of Fmoc-Trp(Boc)-OH prepared in Example 1;
FIG. 5 is an infrared spectrum of Fmoc-Trp(Boc)-OH prepared in Example 1;
FIG. 6 is an HPLC chromatogram of intermediate Fmoc-Trp-OH prepared in Example 2;
FIG. 7 is an HPLC chromatogram of intermediate Fmoc-Trp-OBZl prepared in Example 2;
FIG. 8 is an HPLC chromatogram of intermediate Fmoc-Trp(Boc)-OBZl prepared in Example 2;
FIG. 9 is an HPLC chromatogram of Fmoc-Trp(Boc)-OH prepared in Example 2;
FIG. 10 is an infrared spectrum of Fmoc-Trp(Boc)-OH prepared in Example 2; and
FIG. 11 is a nuclear magnetic resonance spectrum of Fmoc-Trp(Boc)-OH prepared in Example 2.

### DETAILED DESCRIPTION

It should be understood that, the expression "one or more of..." individually includes each object recited after the expression as well as various different combinations of two or more of the recited objects, unless otherwise understood from context and usage. The expression "and/or" in combination with three or more recited objects should be understood to have the same meaning, unless otherwise understood from context.

Use of the terms "comprising", "having", or "containing", including their grammatical synonyms, should generally be understood to be open-ended and non-limiting, for example, not excluding other unrecited elements or steps, unless otherwise specifically stated or otherwise understood from context.

It should be understood that, as long as the present disclosure remains operable, an order of steps or an order of performing certain actions is not critical. Furthermore, two or more steps or actions can be performed concurrently.

Any and all instances or exemplary language herein, such as use of "for example" or "including", is merely intended to better illuminate the disclosure and does not pose a limitation on a scope of the disclosure unless claimed. Any language in this specification should not be construed as indicating any non-claimed element as essential to practice of the disclosure.

Furthermore, numerical ranges and parameters used to define the present disclosure are approximate values, and relevant numerical values in specific embodiments have been presented as precisely as possible herein. However, any numerical value inherently and inevitably contains standard deviations resulting from individual testing methods. Therefore, unless otherwise explicitly stated, it should be understood that all ranges, quantities, numerical values, and percentages used in the present disclosure are modified by "about". Herein, "about" generally means that an actual value is within ±10%, ±5%, ±1%, or ±0.5% of a specific numerical value or range.

The present disclosure provides a method for preparing Fmoc-Trp(Boc)-OH, comprising steps of:
a) reacting tryptophan with N-(9-fluorenylmethoxycarbonyloxy)succinimide in the presence of a base to obtain an intermediate compound as represented by formula (3); 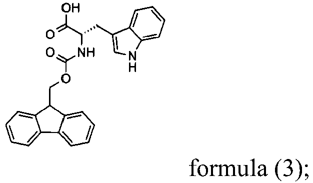
b) reacting the intermediate compound as represented by formula (3) with benzyl halide in the presence of a base to obtain an intermediate compound as represented by formula (4); 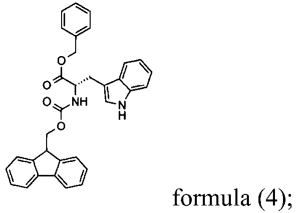
c) reacting the intermediate compound as represented by formula (4) with di-tert-butyl dicarbonate in the presence of a catalyst to obtain an intermediate compound as represented by formula (6); and 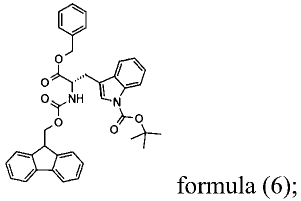
d) deprotecting the intermediate compound as represented by formula (6) to obtain Fmoc-Trp(Boc)-OH.

The present disclosure uses tryptophan (H-Trp-OH) as a starting material to synthesize Fmoc-Trp(Boc)-OH via a four-step reaction comprising a Fmoc introduction reaction, an esterification reaction with benzyl halide, a Boc introduction reaction, and a hydrogenation reaction, avoiding a step of introducing Fmoc on H-Trp(Boc)-OH. The method simplifies the process, features simple operations and mild reaction conditions, and reduces the difficulty of industrial production. Furthermore, the method does not involve expensive raw materials, which is beneficial for controlling production costs, and Fmoc-Trp(Boc)-OH can be obtained with a high purity and a high yield.

The present disclosure, tryptophan (H-Trp-OH) and N-(9-fluorenylmethoxycarbonyloxy)succinimide (Fmoc-Osu) are used as raw materials to react in the presence of a base, to obtain the intermediate Fmoc-Trp-OH as represented by formula (3). The reaction process is as follows:

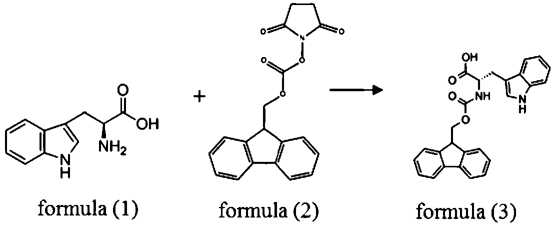

H-Trp-OH has a structure as represented by formula (1), and there is no particular limitation on its source in the present disclosure. Fmoc-Osu has a structure as represented by formula (2), and there is no particular limitation on its source in the present disclosure.

In some specific embodiments, in step a), the base is an inorganic base, which includes but is not limited to sodium carbonate, sodium bicarbonate, potassium carbonate, potassium bicarbonate, sodium hydroxide, potassium hydroxide, and the like, and can be one or more of them. When the base is a combination of multiple substances, there is no particular limitation on the types of them in the present disclosure. In some specific embodiments, the base is selected from the group consisting of sodium carbonate, sodium bicarbonate, and potassium carbonate.

In some specific embodiments, the reaction is performed in a medium, and the medium is water and an organic solvent, wherein the water is used to dissolve tryptophan and the base, and the organic solvent is used to dissolve N-(9-fluorenylmethoxycarbonyloxy)succinimide. There is no particular limitation on the ratio of water and the organic solvent in the present disclosure. The ratio can be determined according to the ratio of tryptophan and N-(9-fluorenylmethoxycarbonyloxy)succinimide, as long as it can provide a medium condition for the reaction. In some specific embodiments, the organic solvent is selected from ethyl acetate, acetone, and tetrahydrofuran.

In some specific embodiments, tryptophan, N-(9-fluorenylmethoxycarbonyloxy)succinimide, and the base have a mole ratio of 1:(0.8 to 1.3):(1 to 2.5), preferably 1:(0.9 to 1.2):(1.5 to 2), more preferably 1:0.96:2.

Specifically, in the present disclosure, water, the base and H-Trp-OH are dissolved first, then the organic solvent and Fmoc-Osu are added, and the reaction is performed to obtain the intermediate compound Fmoc-Trp-OH as represented by formula (3). In some specific embodiments, Fmoc-Osu is added at 25°C to 35°C and the reaction is performed. Adding Fmoc-Osu at this temperature can ensure the complete reaction of Fmoc-Osu and increase the yield of the reaction. In some specific embodiments, the reaction is performed at 25°C to 35°C, preferably 28°C to 32°C, for a duration of 2 h to 6 h, preferably 3 h to 5 h.

After the reaction is completed, the obtained product is subjected to a post-process procedure, specifically comprising steps of:
adding acid to the reaction system to adjust the pH to 2 to 3, separating the layers, and collecting the organic layer;
washing, drying, concentrating, crystallizing, centrifuging, and drying again the organic layer, to obtain the intermediate Fmoc-Trp-OH as represented by formula (3).

There is no particular limitation on the acid in the present disclosure; for example, it can be hydrochloric acid. There is no particular limitation on method of the washing step in the present disclosure; for example, saturated sodium chloride solution can be used for washing 3 times. There is no particular limitation on method of the drying step in the present disclosure; for example, sodium sulfate can be used for drying. There is no particular limitation on method of the crystallizing step in the present disclosure; for example, the system can be cooled to 20°C to 25°C to perform crystallization. There is no particular limitation on method of the drying step after centrifuging in the present disclosure; for example, the drying can be conducted at 50°C to 55°C.

After the intermediate compound as represented by formula (3) is obtained, it is reacted with benzyl halide in the presence of a base to obtain the intermediate as represented by formula (4). Take benzyl bromide as an example, the reaction process is as follows: 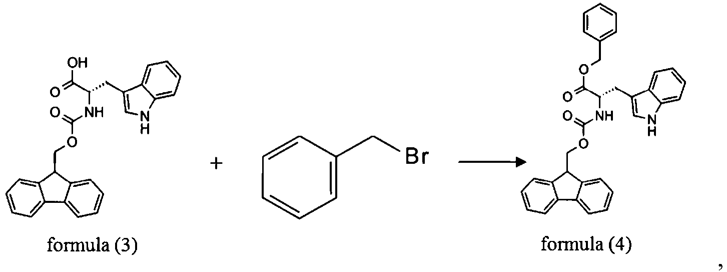 wherein, benzyl halide includes but is not limited to benzyl bromide and benzyl chloride, preferably benzyl bromide. There is no particular limitation on the source of benzyl halide in the present disclosure.

In some specific embodiments, in step b), the base is inorganic base, which includes but is not limited to sodium carbonate, sodium bicarbonate, potassium carbonate, potassium bicarbonate, sodium hydroxide, potassium hydroxide, and the like, and can be one or more of them. When the base is a combination of multiple substances, there is no particular limitation on the types of them in the present disclosure. In some specific embodiments, the base is selected from the group consisting of sodium carbonate and potassium carbonate.

In some specific embodiments, in step b), the reaction is performed in a medium, and the medium is an organic solvent. In some specific embodiments, the organic solvent is selected from the group consisting of acetonitrile and ethyl acetate.

In some specific embodiments, the intermediate compound as represented by formula (3), benzyl halide, and the base have a mole ratio of 1:(1 to 1.5):(1.1 to 2.0), preferably 1:1.1:1.5.

Specifically, in the present disclosure, the intermediate compound as represented by formula (3) and the organic solvent are mixed under stirring first, then the base and benzyl halide are added, and the mixture is heated for reaction under stirring to obtain the intermediate Fmoc-Trp-OBZl as represented by formula (4).

In some specific embodiments, benzyl halide is added at a temperature of 25°C to 30°C, the mixture is heated at a temperature of 60°C to 80°C for reaction for a duration of 2 to 4 h. After adding the benzyl halide, the reaction temperature is maintained at 60°C to 80°C, preferably 70°C to 80°C, which can ensure a quick reaction without generating too many impurities. When the reaction temperature is lower than the aforementioned temperature range, the reaction time is prolonged, the raw materials can not completely react, and the impurities increase.

After the reaction is completed, the obtained product is subjected to a post-process procedure, specifically comprising steps of:
after the reaction is completed, adding water to the reaction system, separating the layers, and collecting the organic layer;
washing, drying, concentrating, crystallizing, centrifuging and drying again the organic layer, to obtain the intermediate Fmoc-Trp-OBZl as represented by formula (4).

There is no particular limitation on method of the washing step in the present disclosure; for example, saturated sodium chloride solution can be used for washing 3 times. There is no particular limitation on method of the drying step in the present disclosure; for example, sodium sulfate can be used for drying. There is no particular limitation on method of the crystallizing step in the present disclosure; for example, crystallization can be performed by cooling the system to 20°C to 25°C and adding a solvent such as petroleum ether. There is no particular limitation on method of the drying step after centrifuging in the present disclosure; for example, drying can be conducted at 50°C to 55°C.

After the intermediate Fmoc-Trp-OBZl as represented by formula (4) is obtained, it is reacted with di-tert-butyl dicarbonate in the presence of a catalyst to obtain the intermediate compound as represented by formula (6). The reaction process is as follows:

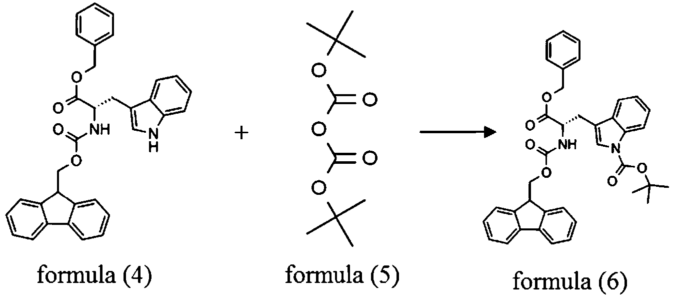

Di-tert-butyl dicarbonate, with a chemical formula of (Boc)₂O and a structural formula as represented by formula (5), reacts with the intermediate compound Fmoc-Trp-OBZl as represented by formula (4) in the presence of a catalyst, to obtain Fmoc-Trp(Boc)-OBZl as represented by formula (6).

In some specific embodiments, in step c), the catalyst is 4-dimethylaminopyridine.

In some specific embodiments, in step c), the reaction is performed in a medium, and the medium is an organic solvent. In some specific embodiments, the organic solvent includes but is not limited to ethyl acetate, dichloromethane, acetonitrile, tetrahydrofuran and the like. Preferably, the organic solvent is selected from the group consisting of acetonitrile, ethyl acetate, dichloromethane, and tetrahydrofuran.

In some specific embodiments, the intermediate represented by formula (4), the catalyst and di-tert-butyl dicarbonate have a mole ratio of 1:(0.040 to 0.045):(1 to 2), preferably 1:0.042:1.2.

Specifically, in the present disclosure, the intermediate compound as represented by formula (4) and the organic solvent are mixed under stirring first, then the catalyst is added, and the reaction is performed under stirring to obtain the intermediate Fmoc-Trp(Boc)-OBZl as represented by formula (6).

In some specific embodiments, the catalyst is added at a temperature of 20°C to 25°C. Adding the catalyst at an excessively high temperature will result in increased impurities. In some specific embodiments, after adding the catalyst, the reaction is performed at a temperature of 10°C to 35°C, preferably 20°C to 25°C, for a duration of 0.5 h to 2 h.

After the reaction is completed, the obtained product is subjected to a post-process procedure, specifically comprising steps of:
after the reaction is completed, adding water to the reaction system, separating the layers, and collecting the organic layer;
washing, drying, and concentrating the organic layer to obtain the intermediate compound Fmoc-Trp(Boc)-OBZl as represented by formula (6).

There is no particular limitation on method of the washing step in the present disclosure; for example, saturated sodium chloride solution can be used for washing 3 times. There is no particular limitation on method of the drying step in the present disclosure; for example, sodium sulfate can be used for drying.

After the intermediate Fmoc-Trp(Boc)-OBZl as represented by formula (6) is obtained, it is deprotected to obtain Fmoc-Trp(Boc)-OH as represented by formula (7). The reaction process is as follows:

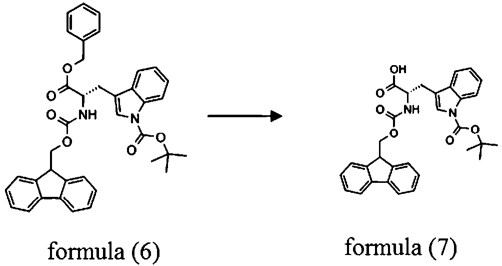

Specifically, in the present disclosure, the deprotection can be performed by a hydrogenation reaction, comprising the steps of:
reacting the intermediate compound represented by formula (6) with hydrogen in the presence of a catalyst to obtain Fmoc-Trp(Boc)-OH.

In some specific embodiments, the catalyst includes but is not limited to Pd/C, raney nickel, Pd/BaSO₄, and the like, preferably Pd/C. In some specific embodiments, the intermediate compound represented by formula (6) and the catalyst have a mass ratio of 1:(0.01 to 0.1), preferably 1:0.05.

In some specific embodiments, in step d), the reaction is performed in a medium, and the medium is an organic solvent. In some specific embodiments, the organic solvent includes but is not limited to methanol, ethanol, ethyl acetate, dichloromethane, and the like, preferably dichloromethane.

Specifically, in the present disclosure, the intermediate compound as represented by formula (6) and the organic solvent are first mixed under stirring, and then the catalyst is added. After purging with nitrogen, hydrogen gas is introduced. The reaction is performed under stirring to obtain Fmoc-Trp(Boc)-OH as represented by formula (7). In some specific embodiments, the reaction is performed at a temperature of 20°C to 30°C for a duration of 16 h to 20 h. An excessive low temperature will result in a prolonged reaction time; and an excessive high temperature will result in increased impurities, which are hard to remove.

After the reaction is completed, the obtained product is subjected to a post-process procedure, specifically comprising steps of:
after the reaction is completed, filtering the reaction system, concentrating the obtained filtrate, adding organic solvent to crystalize, centrifuging, adding organic solvent to the obtained solid and washing under stirring, centrifuging, and drying the obtained solid, to obtain Fmoc-Trp(Boc)-OH.

In some specific embodiments, there is no particular limitation on solvent used in the crystallization step in the present disclosure, for example, it can be a mixture of ethyl acetate and n-hexane. The system can be cooled to 0°C to 5°C for crystallization. There is no particular limitation on solvent used in the washing step in the present disclosure, for example, it can be n-hexane. There is no particular limitation on the drying step after centrifuging in the present disclosure, for example, it can be conducted at a temperature of 15°C to 20°C.

After the reaction is completed, the obtained solid is subjected to HPLC analysis, infrared analysis, and nuclear magnetic resonance analysis. The results indicate that the product is Fmoc-Trp(Boc)-OH, with a purity over 98% and a yield over 75%.

The following examples are provided to further illustrate the method for preparing Fmoc-Trp(Boc)-OH disclosed in the present disclosure.

### Example 1

### (1) Preparation of Fmoc-Trp-OH:

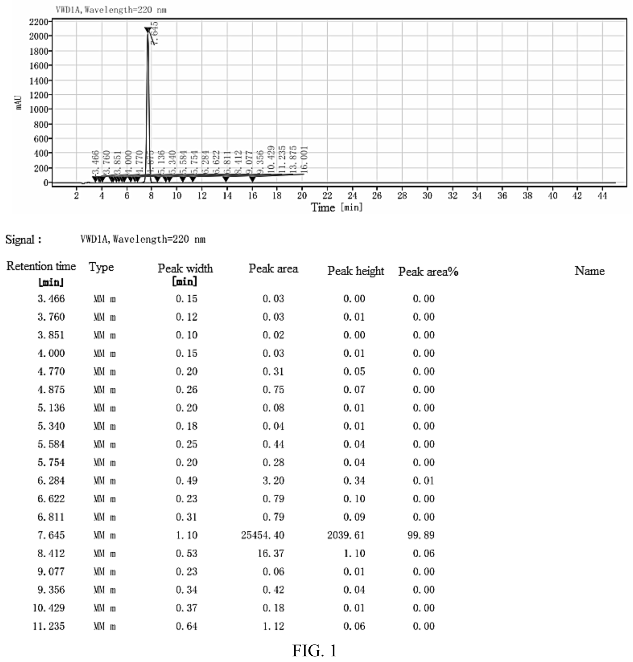

To a 1000 L reaction kettle, 200 kg of purified water was added. Under stirring, 17 kg of sodium carbonate was added, followed by 20 kg of tryptophan (H-Trp-OH) as represented by formula (1). The mixture was stirred until completely dissolved. Subsequently, 270 kg of ethyl acetate was added while the temperature was maintained at 25°C to 30°C. Then, 35 kg of N-(9-fluorenylmethoxycarbonyloxy)succinimide (Fmoc-Osu) as represented by formula (2) was added in one portion. The temperature was maintained at 25°C to 30°C, the pH value was controlled at 8 to 9, and the reaction was performed for 2 h until the raw material H-Trp-OH had completely reacted, as indicated by thin layer chromatography (TLC). Then 48 kg of hydrochloric acid was added to the system to adjust the pH value to 2 to 3. The mixture was subjected to layer separation. The ethyl acetate layer was collected, washed 3 times with saturated sodium chloride solution, dried over sodium sulfate, filtered and concentrated until a large amount of solid precipitated. The mixture was cooled to 20°C to 25°C for crystallization for 2 hours and then centrifuged. The product was dried at 50°C to 55°C to obtain 40.2 kg of a solid intermediate Fmoc-Trp-OH as represented by formula (3), as shown by FIG. 1, which was an HPLC chromatogram of the intermediate Fmoc-Trp-OH prepared in Example 1. The yield was 96.2% and the HPLC purity was 99.89%;

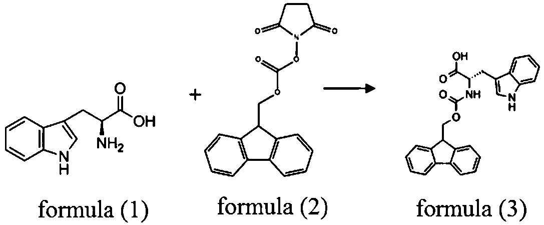

### (2) Preparation of Fmoc-Trp-OBZl:

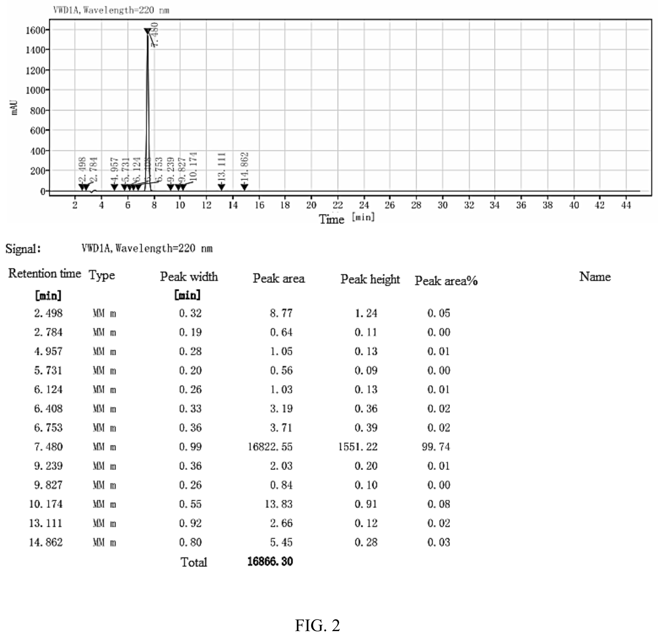

To a clean and anhydrous 2000 L reaction kettle, 402 kg ethyl acetate was added. Under stirring, 40.2 kg of Fmoc-Trp-OH prepared in step (1) was added, followed by 15 kg of sodium carbonate and 17.8 kg of benzyl bromide. Then stirring was initiated. The mixture was heated to an internal temperature of 70°C to 75°C and reacted for 4 h until the raw material Fmoc-Trp-OH was completely reacted, as indicated by TLC. The reaction system was cooled to 20°C to 30°C. Then 500 kg of water was added to the reaction mixture. The mixture was stirred until clear and allowed to stand for layer separation. The ethyl acetate layer was collected, washed 3 times with saturated sodium chloride solution, dried over sodium sulfate, filtered and concentrated until a large amount of solid precipitated. The mixture was cooled to 20°C to 25°C, and 100 kg of petroleum ether was added. The mixture was crystallized for 2 h and centrifuged. The product was dried at 50°C to 55°C to obtain 44.8 kg of the solid intermediate Fmoc-Trp-OBZl, as shown by FIG. 2, which was an HPLC chromatogram of the intermediate Fmoc-Trp-OBZl prepared in Example 1. The yield was 92% and the HPLC purity was 99.74%;

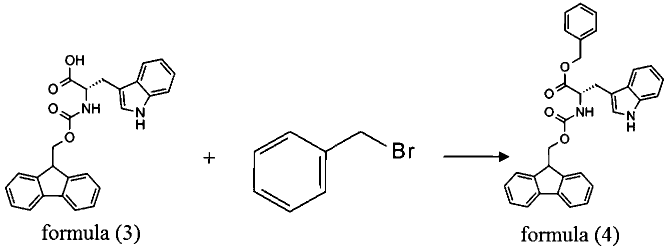

### (3) Preparation of Fmoc-Trp(Boc)-OBZl:

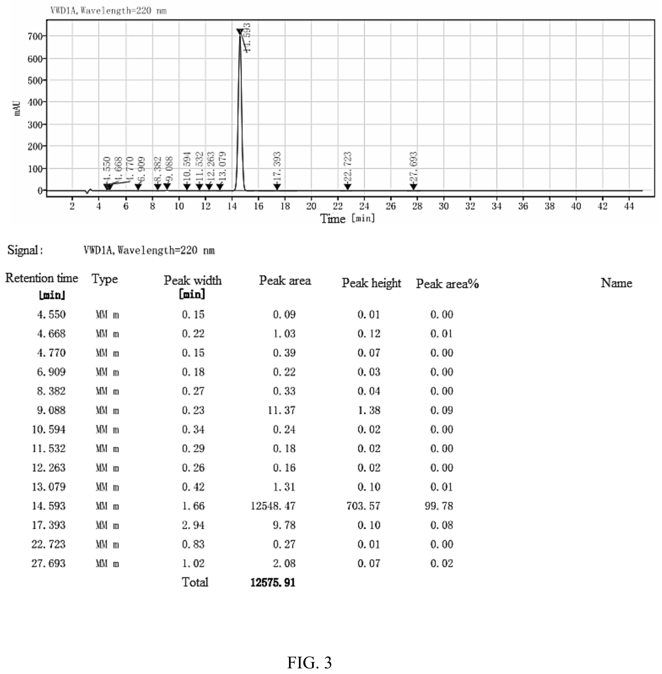

To a clean and anhydrous 2000 L reaction kettle, 448 kg ethyl acetate was added. Under stirring, 44.8 kg of Fmoc-Trp-OBZl prepared in step (2) was added. The mixture was stirred to clear, and 22.7 kg of di-tert-butyl dicarbonate ((Boc)₂O) as represented by formula (5) was added. The temperature was maintained at 20°C to 25°C, 0.45 kg of catalyst 4-dimethylaminopyridine was added, and the reaction was performed at 20°C to 25°C for 2 h until the raw material Fmoc-Trp-OBZl had completely reacted, as shown by TLC. 500 kg of water was added to the system. The mixture was subjected to layer separation. The ethyl acetate layer was collected, washed 3 times with saturated sodium chloride solution, dried over sodium sulfate, filtered and concentrated to dryness, to obtain 50 kg of oily substance intermediate Fmoc-Trp(Boc)-OBZl, as represented by formula (6), as shown by FIG. 3, which was an HPLC chromatogram of the intermediate Fmoc-Trp(Boc)-OBZl prepared in Example 1. The yield was 93.5% and the HPLC purity was 99.78%;

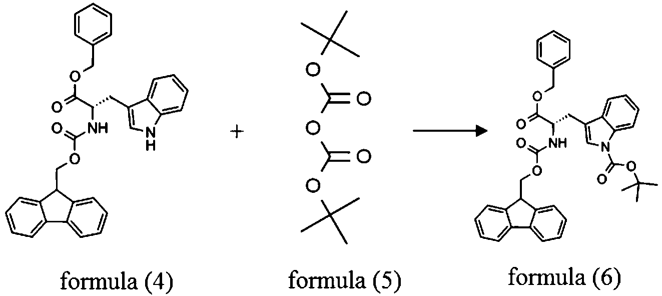

### (4) Preparation of Fmoc-Trp(Boc)-OH:

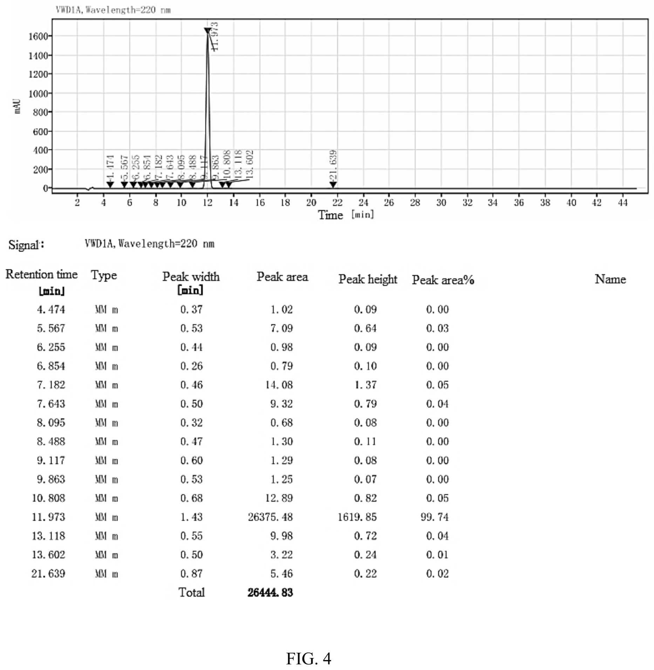

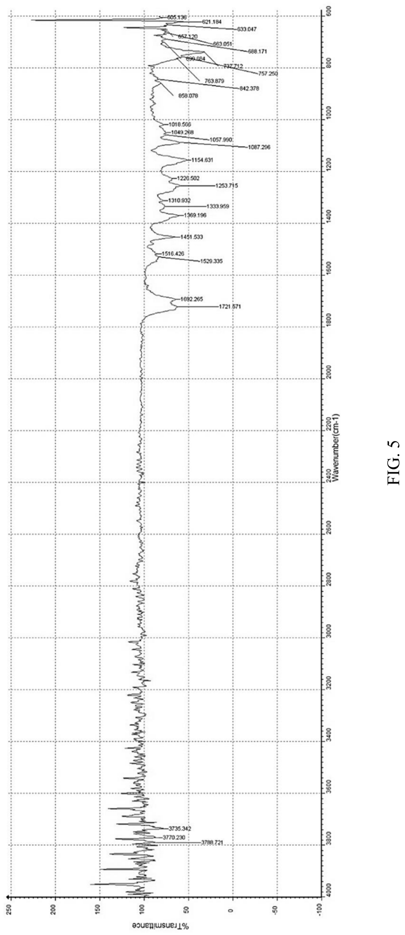

To a 1000 L reaction kettle, 500 kg of dichloromethane and 50 kg of Fmoc-Trp(Boc)-OBZl were added. The mixture was stirred until clear, and 2 kg Pd/C was added. After purging with nitrogen, hydrogen gas was introduced. The temperature was maintained at 25°C to 30°C and the reaction was performed for 15 h until the raw material Fmoc-Trp(Boc)-OBZl had completely reacted, as indicated by TLC. The Pd/C was filtered out and the filtrate was concentrated to dryness. 80 kg of ethyl acetate and 200 kg of n-hexane were added, then the mixture was cooled to 0°C to 5°C for crystallization for 8h and then centrifuged. The precipitated solid was stirred and washed with 70 kg of n-hexane for 2 h and centrifuged. The precipitated solid was dried at 15°C to 20°C to dryness, to obtain 36 kg of solid Fmoc-Trp(Boc)-OH as represented by formula (7), as shown by FIG. 4 and FIG. 5. FIG. 4 is an HPLC chromatogram of Fmoc-Trp(Boc)-OH prepared in Example 1. FIG. 5 is an infrared spectrum of Fmoc-Trp(Boc)-OH prepared in example 1. The yield was 84.3% and the HPLC purity was 99.74%.

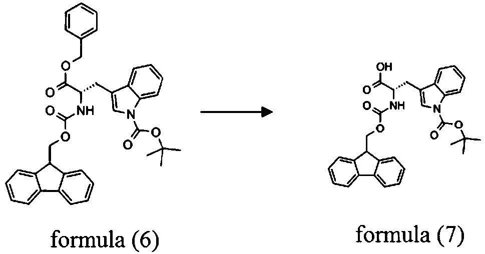

### Example 2

### (1) Preparation of Fmoc-Trp-OH:

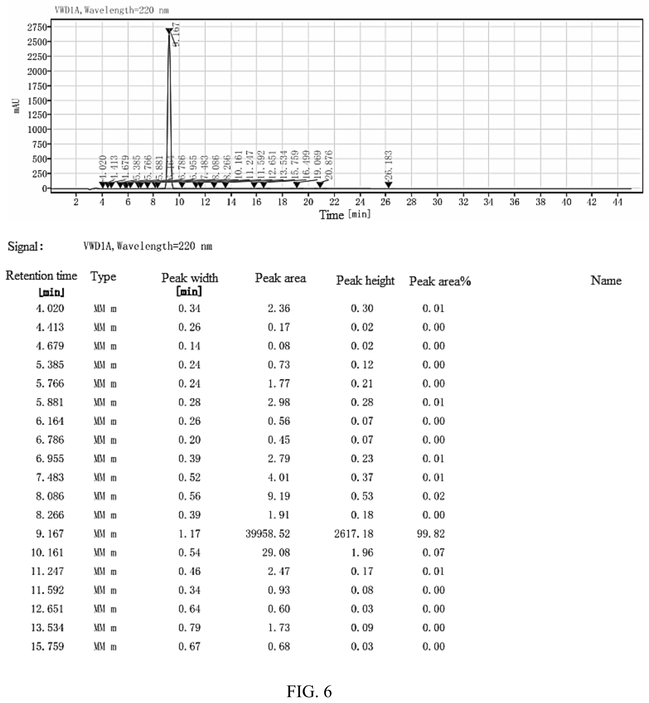

To a 2000 L reaction kettle, 600 kg of purified water was added. Under stirring, 50 kg of sodium carbonate was added, followed by 60 kg of H-Trp-OH. The mixture was stirred until completely dissolved. Subsequently, 800 kg of ethyl acetate was added while the temperature was maintained at 25°C to 30°C. Then, 104 kg of Fmoc-Osu was added in one portion. The temperature was maintained at 25°C to 30°C, the pH was controlled at 8 to 9, and the reaction was performed for 3 h until the raw material H-Trp-OH had completely reacted, as indicated by TLC. Then 150 kg of hydrochloric acid was added to the system to adjust the pH value to 2 to 3. The mixture was subjected to layer separation. The ethyl acetate layer was collected, washed 3 times with saturated sodium chloride solution, dried over sodium sulfate, filtered and concentrated until a large amount of solid precipitated. The mixture was cooled to 20°C to 25°C for crystallization for 2 hours and then centrifuged. The product was dried at 50°C to 55°C to obtain 120 kg of a solid intermediate Fmoc-Trp-OH, as shown by FIG. 6, which was an HPLC chromatogram of the intermediate Fmoc-Trp-OH prepared in Example 2. The yield was 95.7% and the HPLC purity was 99.82%;

### (2) Preparation of Fmoc-Trp-OBZl:

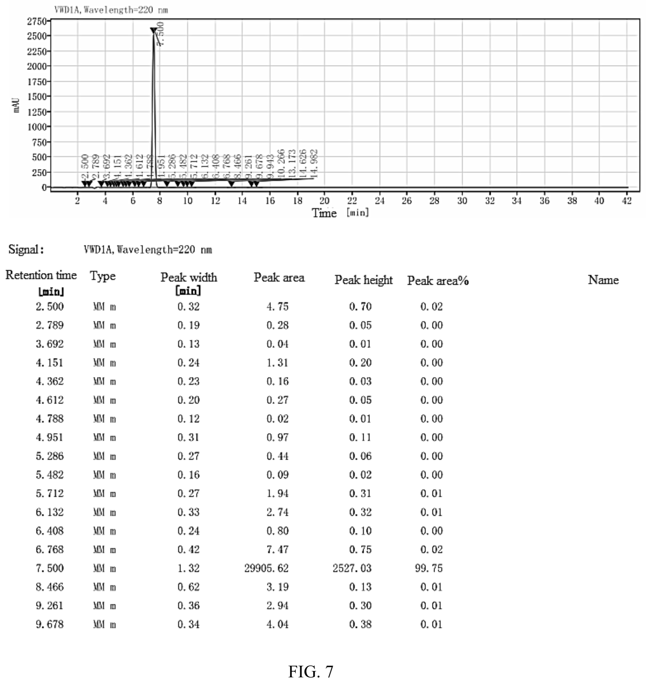

To a clean and anhydrous 3000 L reaction kettle, 1200 kg acetonitrile was added. Under stirring, 120 kg of Fmoc-Trp-OH prepared in step (1) was added, followed by 45 kg of sodium carbonate and 39 kg of benzyl chloride. Then, mixing was initiated. The mixture was heated to an internal temperature of 70°C to 75°C and reacted for 4 h until the raw material Fmoc-Trp-OH was completely reacted, as indicated by TLC. The reaction system was cooled to 20°C to 30°C. Then 500 kg of water and 1000 kg ethyl acetate was added to the reaction mixture. The mixture was stirred until clear and allowed to stand for layer separation. The ethyl acetate layer was collected, washed 3 times with saturated sodium chloride solution, dried over sodium sulfate, filtered and concentrated until a large amount of solid precipitated. The mixture was cooled to 20°C to 25°C, and 300 kg of petroleum ether was added. The mixture was crystallized for 2 h and centrifuged. The product was dried at 50°C to 55°C to obtain 133 kg of the solid intermediate Fmoc-Trp-OBZl, as shown by FIG. 7, which was an HPLC chromatogram of the intermediate Fmoc-Trp-OBZl prepared in Example 2. The yield was 91.5% and the HPLC purity was 99.75%;

### (3) Preparation of Fmoc-Trp(Boc)-OBZl:

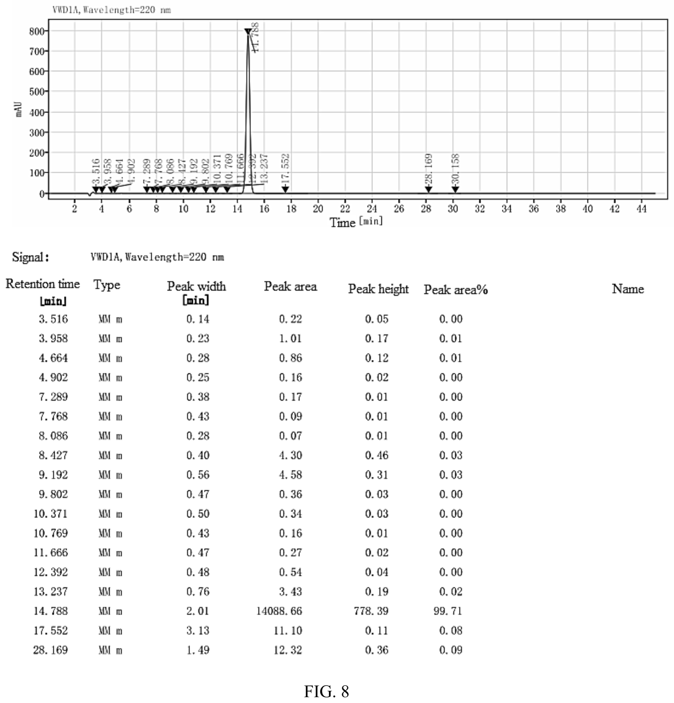

To a clean and anhydrous 3000 L reaction kettle, 1330 kg tetrahydrofuran was added. Under stirring, 133 kg of Fmoc-Trp-OBZl prepared in step (2) was added. The mixture was stirred to clear, and 53 kg of (Boc)₂O was added. The temperature was maintained at 20°C to 25°C, 1.33 kg of 4-dimethylaminopyridine was added, and the reaction was performed at 20°C to 25°C for 3 h until the raw material Fmoc-Trp-OBZl had completely reacted, as shown by TLC. 500 kg of water was added to the system, followed by 500 kg of ethyl acetate for extraction. The mixture was subjected to layer separation. The ethyl acetate layer was collected, washed 3 times with saturated sodium chloride solution, dried over sodium sulfate, filtered and concentrated to dryness, to obtain 155.6 kg of oily substance intermediate Fmoc-Trp(Boc)-OBZl, as shown by FIG. 8, which was an HPLC chromatogram of the intermediate Fmoc-Trp(Boc)-OBZl prepared in Example 2. The yield was 98% and the HPLC purity was 99.71%;

### (4) Preparation of Fmoc-Trp(Boc)-OH:

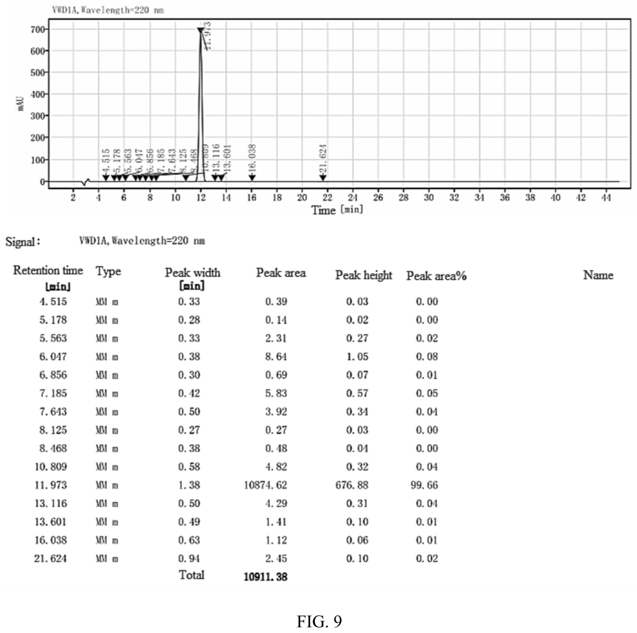

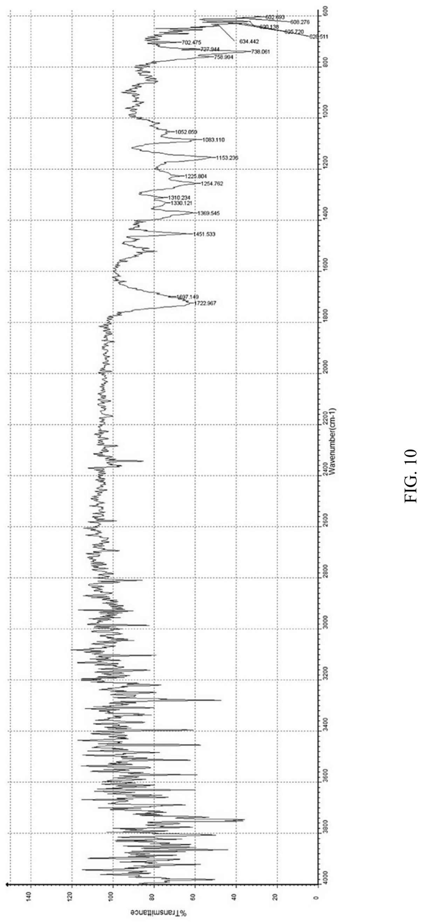

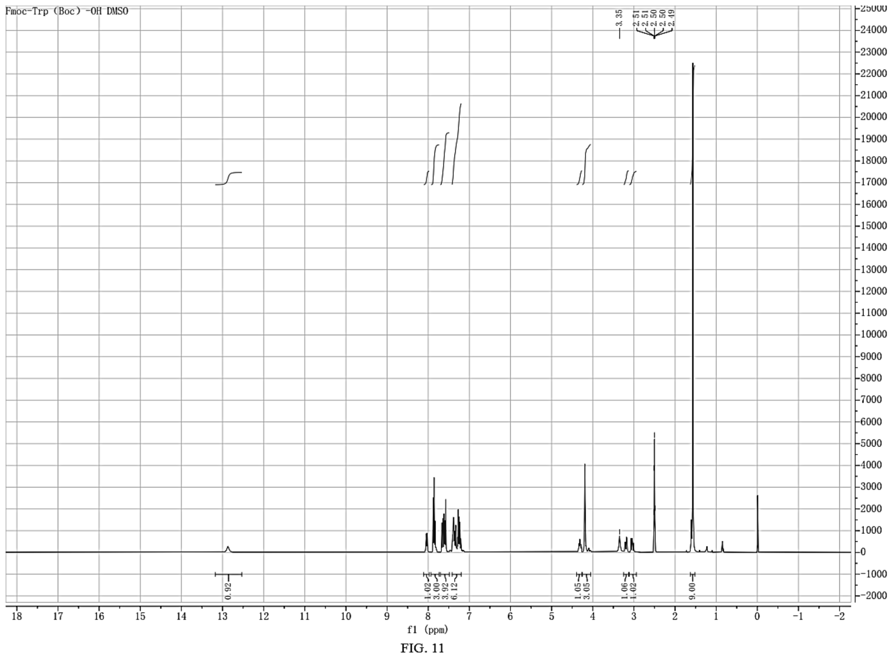

To a 3000 L reaction kettle, 1556 kg of ethyl acetate, and 155.6 kg of Fmoc-Trp(Boc)-OBZl were added. The mixture was stirred until clear, and 7.78 kg Pd/C was added. After purging with nitrogen, hydrogen gas was introduced. The temperature was maintained at 30°C to 35°C and the reaction was performed for 18 h until the raw material Fmoc-Trp(Boc)-OBZl had completely reacted, as indicated by TLC. The Pd/C was filtered out and the filtrate was concentrated to a remaining volume of about 300 L. 600 kg of n-hexane was added, then the mixture was cooled to 0°C to 5°C for crystallization for 8h and then centrifuged. The precipitated solid was stirred and washed with 200 kg of n-hexane for 2 h and centrifuged. The precipitated solid was dried at 15°C to 20°C to dryness, to obtain 118 kg of solid Fmoc-Trp(Boc)-OH, as shown by FIG. 9, FIG. 10 and FIG. 11. FIG. 9 is an HPLC chromatogram of Fmoc-Trp(Boc)-OH prepared in example 2; FIG. 10 is an infrared spectrum of Fmoc-Trp(Boc)-OH prepared in example 2; FIG. 11 is a nuclear magnetic resonance spectrum of Fmoc-Trp(Boc)-OH prepared in example 2. The yield was 88.8% and the HPLC purity was 99.66%.

### Examples 3 to 4

These examples investigated the influence of the inorganic base in step (1) on the intermediate Fmoc-Trp-OH. Specifically, the preparation steps and technical parameters in the preparation method of Examples 3-4 were the same as those in step (1) of Example 1, except that the type of the inorganic base was changed. The results of step (1) were shown in Table 1.

**Table 1**

| | Inorganic Base | Reaction Time | Yield | Purity |
|---|---|---|---|---|
| Example 1 | sodium carbonate | 2 h to 3 h | 96.2% | 99.89% |
| Example 3 | sodium bicarbonate | 10 h to 12 h | 93.8% | 99.57% |
| Example 4 | potassium carbonate | 1 h to 2 h | 95.2% | 98.68% |

### Example 5 and Comparative Examples 1 to 4

These example and comparative examples investigated the influence of the inorganic base in step (2) on the intermediate Fmoc-Trp-OBZl. Specifically, the preparation steps and technical parameters in the preparation method of Example 5 and Comparative Examples 1 to 4 were the same as those in step (2) of Example 1, except that the type of the inorganic base was changed. Through tests, the purities and yields of Fmoc-Trp-OBZl were shown in Table 2 below.

**Table 2**

| | Inorganic Base | Reaction Time | Yield | Purity |
|---|---|---|---|---|
| Example 1 | sodium carbonate | 4 h | 92% | 99.74% |
| Example 5 | potassium carbonate | 4 h | 93.7% | 98.68% |
| Comparative Example 1 | sodium bicarbonate | 28 h | 88.4% | 96.27% |
| Comparative Example 2 | potassium hydroxide | 3 h | 93.2% | 93.14% |
| Comparative Example 3 | potassium bicarbonate | 20 h | 89.5% | 95.53% |
| Comparative Example 4 | sodium hydroxide | 3 h | 92.8% | 94.11% |

### Examples 6 to 7 and Comparative Examples 5 to 6

These examples and comparative examples investigated the influence of reaction temperature in step (2) on the intermediate Fmoc-Trp-OBZl. Specifically, the preparation steps and technical parameters in the preparation method of Examples 6 to 7 and Comparative Examples 5 to 6 were the same as those in step (2) of Example 1, except that the reaction temperature was changed. Through tests, the yields and reaction time of Fmoc-Trp-OBZl were shown in Table 3 below.

**Table 3**

| | Reaction Temperature | Reaction Time | Yield | Purity |
|---|---|---|---|---|
| Example 6 | 76°C to 80°C | 4 h | 90% | 99.35% |
| Example 1 | 70°C to 75°C | 4 h | 92% | 99.74% |
| Example 7 | 65°C to 69°C | 8 h | 85% | 98.45% |
| Comparative Example 5 | 50°C to 55°C | 14 h | 63% | 96.32% |
| Comparative Example 6 | 30°C to 35°C | failed to completely react after 48 h | 36% | 88.5% |

### Example 8 and Comparative Example 7

These example and comparative example investigated the influence of the organic solvent in step (2) on the intermediate Fmoc-Trp-OBZl. Specifically, the preparation steps and technical parameters in the preparation method of Example 8 and Comparative Example 7 were the same as those in step (2) of Example 1, except that the type of the organic solvent was changed. Through tests, the yields and purities of Fmoc-Trp-OBZl were shown in Table 4 below.

**Table 4**

| | Organic Solvent | Yield | Purity |
|---|---|---|---|
| Example 1 | ethyl acetate | 92% | 99.74% |
| Example 8 | acetonitrile | 91% | 99.68% |
| Comparative Example 7 | tetrahydrofuran | 63% | 96.43% |

### Examples 9 to 10

These examples investigated the influence of the organic solvent in step (3) on the intermediate Fmoc-Trp(Boc)-OBZl. Specifically, the preparation steps and technical parameters in the preparation method of Examples 9-10 were the same as those in step (3) of Example 1, except that the type of the organic solvent was changed. Through tests, the yields and purities of Fmoc-Trp(Boc)-OBZl were shown in Table 5 below.

**Table 5**

| | Organic Solvent | Yield | Purity |
|---|---|---|---|
| Example 1 | ethyl acetate | 93.5% | 99.78% |
| Example 9 | acetonitrile | 91% | 99.36% |
| Example 2 | tetrahydrofuran | 98% | 99.71% |
| Example 10 | dichloromethane | 94.5% | 99.45% |

### Examples 11 to 14 and Comparative Example 8

These examples and comparative example investigated the influence of reaction temperature in step (4) on the intermediate Fmoc-Trp(Boc)-OH. Specifically, the preparation steps and technical parameters in the preparation method of Examples 11 to 14 and Comparative Example 8 were the same as those in step (4) of Example 1, except that the reaction temperature was changed. Through tests, the purities and reaction time of Fmoc-Trp(Boc)-OH were shown in Table 6 below.

**Table 6**

| | Reaction Temperature | Purity | Reaction Time | Yield |
|---|---|---|---|---|
| Example 1 | 25°C to 30°C | 99.74% | 15 h | 84.3% |
| Example 11 | 20°C to 24°C | 99.42% | 16 h | 82.1% |
| Example 12 | 15°C to 19°C | 99.23% | 23 h | 81.2% |
| Example 13 | 10°C to 14°C | 99.11% | 33 h | 76.8% |
| Example 14 | 31°C to 35°C | 98.58% | 13 h | 83.1% |
| Comparative Example 8 | 36°C to 40°C | 97.69% | 10 h | 80.8% |

In summary, the present disclosure provides a method for preparing Fmoc-Trp(Boc)-OH, which is suitable for scaling-up production. The synthesis method features simple equipment requirements, good reaction efficiency, and a relatively low production cost. Moreover, the final product can be obtained with a purity over 98.0% and a yield over 75%.

The foregoing descriptions are merely preferred embodiments of the present disclosure. It should be noted that for those of ordinary skill in the art, several improvements and embellishments can be made without departing from the principles of the present disclosure. These improvements and embellishments should also be considered within the scope of protection of the present disclosure.

## Claims

1. A method for preparing Fmoc-Trp(Boc)-OH, comprising steps of:
a) reacting tryptophan with N-(9-fluorenylmethoxycarbonyloxy)succinimide in the presence of a base to obtain an intermediate compound as represented by formula (3); 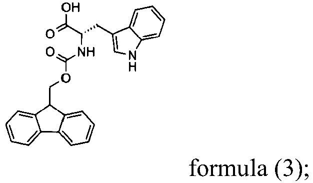
b) reacting the intermediate compound as represented by formula (3) with benzyl halide in the presence of a base to obtain an intermediate compound as represented by formula (4); 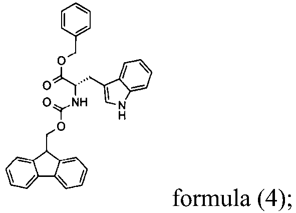
c) reacting the intermediate compound as represented by formula (4) with di-tert-butyl dicarbonate in the presence of a catalyst to obtain an intermediate compound as represented by formula (6); and 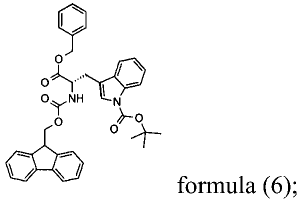
d) deprotecting the intermediate compound as represented by formula (6) to obtain Fmoc-Trp(Boc)-OH.

2. The method according to claim 1, wherein in step a), the base is an inorganic base; and
tryptophan, N-(9-fluorenylmethoxycarbonyloxy)succinimide, and the base have a mole ratio of 1:(0.8 to 1.3):(1 to 2.5).

3. The method according to claim 2, wherein in step a), the reaction is performed at 25°C to 35°C for 2 h to 6 h; and
the reaction is performed in a medium, and the medium is water and an organic solvent, wherein the organic solvent is selected from the group consisting of ethyl acetate, acetone, and tetrahydrofuran.

4. The method according to claim 1, wherein in step b), the base is an inorganic base; and
the intermediate compound as represented by formula (3), benzyl halide, and the base have a mole ratio of 1:(1 to 1.5):(1.1 to 2.0).

5. The method according to claim 4, wherein in step b), the base is selected from the group consisting of sodium carbonate and potassium carbonate.

6. The method according to claim 4, wherein in step b), the reaction is performed at 60°C to 80°C for 2 h to 4 h; and
the reaction is performed in a medium, and the medium is an organic solvent, wherein the organic solvent is selected from the group consisting of acetonitrile and ethyl acetate.

7. The method according to claim 1, wherein in step c), the catalyst is 4-dimethylaminopyridine; and
the intermediate compound as represented by formula (4), the catalyst, and di-tert-butyl dicarbonate have a mole ratio of 1:(0.040 to 0.045):(1 to 2).

8. The method according to claim 7, wherein in step c), the reaction is performed at 10°C to 35°C for 0.5 h to 2 h; and
the reaction is performed in a medium, and the medium is an organic solvent, wherein the organic solvent is selected from the group consisting of dichloromethane, acetonitrile, ethyl acetate, and tetrahydrofuran.

9. The method according to claim 1, wherein step d) specifically comprises:
reacting the intermediate compound as represented by formula (6) with hydrogen in the presence of a catalyst to obtain Fmoc-Trp(Boc)-OH.

10. The method according to claim 9, wherein in step d), the catalyst is selected from the group consisting of Pd/C, raney nickel, and Pd/BaSO₄;
the intermediate compound as represented by formula (6) and the catalyst have a mass ratio of 1:(0.01 to 0.1); and
the reaction is performed at 20°C to 30°C for 16 h to 20 h.
